# EUROPEAN PATENT SPECIFICATION

(11) **EP 4 181 883 B1**
(45) Date of publication and mention of the grant of the patent: **19.11.2025**
(21) Application number: 22802040.0
(22) Date of filing: 07.10.2022
(51) Int. Cl.: A61K 9/14, A61K 9/20, A61K 31/496

(54) **GRANULES CONTAINING POSACONAZOLE**
POSACONAZOL-HALTIGE GRANULATE
GRANULES CONTENANT DU POSACONAZOLE

(30) Priority: 25.11.2021 IN 202111054471; 14.02.2022 EP 22156597
(43) Date of publication of application: 24.05.2023
(73) Proprietor: Alfred E. Tiefenbacher (GmbH & Co. KG), 22767 Hamburg (DE)
(72) Inventor: VELCHURI, Srikanth, 502319 Telangana (IN); PRATHAP, Vamshi Ramana, 502319 Telangana (IN); KALAMATA, Venkatasimhadri Naidu, 22767 Hamburg (DE); ZOUBARI, Gaith, 22767 Hamburg (DE); FITZNER, Angar, 22767 Hamburg (DE); RALLABANDI, Bala Ramesha Chary, 502319 Telangana (IN); MADALLAPALLI, Kiran Kumar, 502319 Telangana (IN)
(74) Representative: Hamm&Wittkopp Patentanwälte PartmbB
(86) International application number: PCT/EP2022/077973
(87) International publication number: WO 2023/012378

(56) References cited:
- WO-A1-2017/025292
- WO-A2-2009/129300
- WO-A2-2009/129301
- WO-A2-2019/240698
- CN-A- 108 125 921

## Description

I he present invention relates to granules comprising posaconazole, a process for preparing granules comprising posaconazole, and a gastro-resistant, optionally film-coated tablet prepared from granules comprising posaconazole.

Posaconazole is a triazole antifungal drug marketed under the tradename Noxafil^{®} as a concentrate for solution for infusion, oral suspension and gastro-resistant tablet for the treatment and prophylaxis of invasive fungal infections. Noxafil^{®} is in particular indicated for the prophylaxis of invasive *Aspergillus* and *Candida* infections in severely immunocompromised patients, such as hematopoietic stem cell transplant recipients with a graft-versus-host-disease and patients with hematologic malignancies with prolonged neutropenia from chemotherapy. The oral suspension is also indicated for the treatment of oropharyngeal candidiasis.

Posaconazole is a white powder with a low aqueous solubility, whereby posaconazole's bioavailability in the oral suspension is significantly enhanced when coad-ministered with food. For this reason, the oral suspension should be administered during or immediately following a full meal to enhance the oral absorption of the drug. The gastro-resistant tablet has an improved bioavailability and can be administered without regard to food.

It is commonly known that the dissolution behaviour of a drug depends on its solid state. Different crystalline forms of a drug usually exhibit different dissolution profiles. whereby amorphous forms are generally much more soluble than their crystalline counterparts. In addition, the chemical and physical stability of a drug are dependent on the solid state. Quite often, metastable crystalline or amorphous forms of a drug have to be stabilized in the pharmaceutical composition in order to prevent chemical degradation and interconversion of the crystalline forms/recrystallization of the amorphous form and, thus, fluctuations in the bioavailability.

WO 99/18097 discloses the crystalline forms I, II and III of posaconazole, Form I is the most stable form that does not convert into any other crystalline form under normal storage conditions or under specific stress conditions. The crystalline forms II and III convert into the form I at temperatures between 100°C and 125°C.

WO 2009/147075 discloses the crystalline form Y of posaconazole. The form Y is as stable as form I but has a better water solubility, which results in an improved bioavailability.

WO 2010/000668 reports that the crystalline form IV of posaconazole has a better stability in an aqueous suspension and a better water solubility as form I because of a smaller particle size and, thus, larger specific surface area. The crystalline form IV can be directly used for a pharmaceutical composition, i.e. without the need of reducing the particle size by micronization.

WO 2011/158248 discloses the crystalline form V of posaconazole. while WO 2011/003992 discloses the crystalline form II-S from which the other crystalline forms, in particular the crystalline form IV may be obtained.

As an alternative approach for overcoming the solubility problems encountered with posaconazole, WO 98/00113 suggests a pharmaceutical composition comprising a solid solution of the drug within a polymer. The solid solution is prepared by dissolving the drug and a soluble polymer in a suitable organic solvent, followed by removing the solvent, or by dissolving the drug in a suitable organic solvent and adding an insoluble polymer, followed by absorbing the solution into the insoluble polymeric matrix. Preferably, the polymer is povidone or crospovidone.

WO 2009/129301 discloses a solid solution of posaconazole within hydroxypropyl methylcellulose acetate succinate (HPMCAS) by spray-drying a solution containing the drug and the polymer. It is further suggested that the solid solutions may be prepared by using hot-melt extrusion.

WO 2009/129300 discloses the preparation of a solid solution containing posaconazole within a hydroxypropyl methylcellulose derivative, preferably HPMCAS. It has been found that posaconazole forms a solution with the polymer behaving as a eutectic having a melting point below the melting point of the drug (about 169°C). Hence, the use of hydroxypropyl methylcellulose derivatives for the preparation of the solid solution minimizes thermal decomposition and oxidation of posaconazole during the preparation compared to processes which utilize higher melting polymers. WO 2009/129300 further suggests that the solid solution may additionally contain a plasticizer and an antioxidant.

EP 3 006 049 discloses a solid solution of posaconazole within HPMCAS prepared by hot-melt extrusion. It was found that the use of a specific HPMCAS, which has a hydroxypropyl molar substitution of at least 0.40 and a mole ratio of acetyl to succinyl of less than 1.6 allows the use of lower temperatures in the hot-melt extrusion process.

EP 3 130 354 suggests the use of poly(vinylpyrrolidone/vinylacetate) or a polymer containing ethylene glycol units, e.g. polyvinylcaprolactam/polyvinylacetate/polyethylene glycol graft copolymer, for preparing a solid dispersion containing posaconazole.

US 2015/0231081 suggests a solid solution containing posaconazole dispersed in a polymer other than a hydroxypropyl methylcellulose derivative, such as polyvinylpyrrolidone and poly(methacrylic acid/ethyl acrylate).

WO 2017/032908 discloses a solid dispersion of posaconazole within at least two different enteric polymers, whereby the solid dispersion is prepared by wet-granulation. Preferably, the two different enteric polymers are selected from HPMCAS poly(methacrylic acid/methyl methacrylate) and poly(methacrylic acid/ethyl acrylate).

EP 3 342 399 discloses the use of enteric-coated granules containing posaconazole dispersed within a polymer matrix for the preparation of a unit dosage form, preferably a tablet. A suitable matrix polymer is HPMCAS.

WO 2019/240698 discloses the preparation of a solid dispersion containing posaconazole, an enteric polymer and a surfactant.

WO 2017/025292 relates to a gastro-resistant pharmaceutical composition comprising posaconazole molecularly dispersed in a mixture containing an enteric polymer and a non-enteric polymer, whereby the mixture is prepared by hot-melt extrusion, and whereby the composition contains an antioxidant.

WO 2019/240698 relates to a pharmaceutical composition comprising posaconazole and a dispersion carrier consisting of an enteric polymer, optionally a non-enteric polymer and optionally a surfactant.

WO 2020/159562 relates to a gastro-resistant pharmaceutical composition comprising posaconazole molecularly dispersed in a mixture containing HPMCAS and hydroxypropylcellulose.

It was found that the gastro-resistant unit dosage forms described in the state of the art, in particular the tablets show a certain variability in the dissolution behaviour of the drug, which cannot be explained by differences in the particle size of the drug because the tablet contains posaconazole in molecularly dispersed form. The variability in the dissolution behaviour may cause fluctuations in the bioavailability.

The problem underlying the present invention was the provision of a gastro-resistant unit dosage form containing posaconazole that does not show variability in the dissolution behaviour of the drug and that achieves sufficient bioavailability. This problem has been solved by the subject matter as defined in the claims.

The unit dosage form of the present invention is a gastro-resistant, optionally film-coated tablet prepared from granules containing posaconazole molecularly dispersed in a gastro-resistant matrix. Gastro-resistant formulations are designed to release the drug in the intestines. According to the European Pharmacopoeia 10.0, gastro-resistant unit dosage forms are delayed-release unit dosage forms that are intended to resist the gastric fluid and to release their drug(s) in the intestinal fluid. The gastro-resistance minimizes the food effect of the unit dosage form of the present invention and, thus, improves the bioavailability of the drug.

The gastro-resistant unit dosage form of the present invention is an optionally film-coated tablet that contains posaconazole molecularly dispersed in a mixture containing an enteric polymer and optionally a non-enteric polymer, wherein the mixture is preferably prepared by hot-melt extrusion, and wherein the weight ratio of the enteric polymer to posaconazole is in the range of 5 : 1 to 1 : 1, preferably 5 : 1 to 1.5 : 1, more preferred 4 : 1 to 1 : 1, even more preferred 3.3 : 1 to 1.5 : 1, and most preferably 2.8 : 1 to 1.8 : 1. A process such as hot-melt extrusion provides the mixture in the form of an extrudate that needs to be milled to granules in order to be compressed into the tablet. Alternatively the granules may be prepared by wet-granulation, typically by spray-granulation/spray-drying, e.g. by using a fluid bed process. It was found that at least 10 wt.-% of the granules, but not more than 80 wt.-%, should have a particle size of 250 µm or more, as determined by sieve analysis, or Raman mapping, or SEM-EDX (as described, e.g., in International Journal of Pharmaceutics 2014, 470, 88-98), or dynamic light scattering (DLS) with Mastersizer 2000 (dry method), in order to minimize variability in the dissolution behaviour. It was found that the dissolution of the tablet is too slow in the buffer stage (phosphate buffer. pH 6.8). if more than 80 wt.-% of the granules have a particle size of 250 µm or more, and too fast in the acid stage (0.01 N hydrochloric acid), if less than 10 wt.-% of the granules have a particle size of 250 µm or more. The European Pharmacopoeia 10.0 describes, to show gastro-resistance of a tablet, a suitable dissolution test in general chapter 2.9.3. Dissolution test for solid dosage forms'. In this test, the gastro-resistant tablets are exposed for 2 hours to an acid medium (called 'acid stage'; typically 0.1 M hydrochloric acid), and afterwards a buffer (typically phosphate buffer) is added and the pH is raised (typically to pH 6.8; called 'buffer stage'). The requirement for a gastro-resistant unit dosage form is 10% or less dissolution of the drug in the acid stage, and not less than 85% dissolution of the drug in the buffer stage within 15 minutes. Specifically for posaconazole and 100 mg posaconazole delayed-release tablets, the U.S. FDA requires in its official dissolution methods database as media 0.01 N HCl in the acid stage and 50 mM phosphate buffer having pH 6.8 in the buffer stage.

Thus, at least 10 wt.-%, preferably at least 20 wt.-%, more preferred at least 30 wt.-% and even more preferred at least 40 wt.-% of the granules of the present invention, but not more than 80 wt.-%, preferably not more than 70 wt.-%, more preferred not more than 60 wt.-%, and even more preferred not more than 55 wt-%, have a particle size of 250 µm or more, as determined by sieve analysis. In a preferred embodiment of the invention, at least 30 wt.-%, preferably at least 40 wt.-%. but not more than 70 wt.-%. preferably not more than 55 wt.-% of the granules have a particle size of 250 µm or more, as determined by sieve analysis.

Preferred embodiments of the granules and the tablet of the present application are described in dependent claims.

According to an embodiment of the invention, d (0.1) of the granules is at least 60 µm, preferably at least 75 µm, and more preferred at least 100 µm, but preferably is not more than 250 µm, more preferably not more than 180 µm, and even more preferred not more than 130 µm.

According to another embodiment of the invention, d (0.5) of the granules is at least 160 µm, preferably at least 200 µm, and more preferred at least 250 µm, but preferably is not more than 500 µm, more preferably not more than 400 µm, and even more preferred not more than 320 µm.

According to another embodiment of the invention, d (0.9) of the granules is at least 300 µm, preferably at least 400 µm, and more preferred at least 480 µm, but preferably is not more than 1000 µm, more preferably not more than 800 µm, and even more preferred not more than 600 µm.

The term d (0.1 ) means the size at which 10% by volume of the granules are finer and d (0.5) means the size at which 50% by volume of the granules are finer and d (0.9) means the size at which 90% by volume of the granules are finer. Said values are typically measured by means of DLS, e.g. with a Mastersizer 2000, (dry method).

The maximum particle size of the granules of the invention in general is 3000 µm, preferably 2000 µm, more preferred 1500 µm, and even more preferred 1000 µm.

The enteric polymer is preferably selected from hypromellose derivatives, cellulose derivatives, polyvinylacetate derivatives and polymethacrylic acid derivatives.

The hypromellose derivatives are selected from hydroxypropyl methylcellulose phthalate (hypromellose phthalate, HPMCP, e.g. available as HP-50 or HP-55 from Shin-Etsu Chemical Co., Ltd. Japan), hydroxypropyl methylcellulose succinate and hydroxypropyl methylcellulose acetate succinate (hypromellose acetate succinate. HPMCAS, e.g. available as AQOAT^{®} from Shin-Etsu Chemical Co., Ltd. Japan). The polyvinylacetate derivative is polyvinylacetate phthalate (PVAP), while the cellulose derivatives are selected from cellulose acetate phthalate (CAP), cellulose acetate terephthalate, cellulose acetate isophthalate, cellulose acetate butyrate (CAB), cellulose acetate trimellitate (CAT), methylcellulose acetate phthalate and hydroxypropylcellulose acetate phthalate. The polymethacrylic acid derivatives are selected from poly(methacrylic acid/methyl methacrylate) 1:1 (e.g. available as Eudragit^{®} L 100 from Evonik, Germany), poly(methacrylic acid/methyl methacrylate) 1:2 (e.g. available as Eudragit^{®} S 100 from Evonik, Germany) and poly(methacrylic acid/ethyl acrylate) (Methacrylic Acid/Ethyl Acrylate Copolymer (1:1) Type B, e.g. available as Kollicoat^{®} MAE from BASF SE, Germany). According to a preferred embodiment of the present invention, the enteric polymer is HPMCAS, poly(methacrylic acid/methyl methacrylate) and/or poly(methacrylic acid/ethyl acrylate), and most preferably poly(methacrylic acid/ethyl acrylate) or HPMCAS.

The granules of the present invention may contain a non-enteric polymer that is preferably selected from polyvinylpyrrolidone (povidone), poly(vinylpyrrolidone/ vinylacetate) (e.g. copovidone), polyvinylcaprolactam/polyvinylacetate/polyethylene glycol graft copolymer, polyethylene glycol/polyvinyl alcohol graft copolymer, polyethylene oxide, polypropylene oxide, copolymers of ethylene oxide and propylene oxide, macrogolglycerol hydroxystearate, polyethylene glycol and a water soluble neutral or anionic polysaccharide, wherein a water soluble neutral or anionic polysaccharide is particularly preferred.

Preferably, the water soluble neutral or anionic polysaccharide is selected from a gum, pectin, dextran, dextrin, a cellulose ether, pregelatinized starch and starch ether Examples of suitable gums include xanthan gum, guar gum, gellan gum, acacia gum, agar-agar gum, locust bean gum, karaya gum, carrageenan, galageenan, pullulan, tragaeanth, alginic acid and sodium alginate. Examples of a suitable water soluble cellulose ether include hydroxyalkyl cellulose, methylcellulose, methyl ethylcellulose, sodium carboxymethylcellulose (NaCMC) and carboxymethyl ethylcellulose (CMEC). Preferably, the hydroxyalkyl cellulose is selected from hydroxypropyl methylcellulose (HPMC or hypromellose), hydroxyethyl methylcellulose (HEMC or hymetellose), hydroxypropylcellulose (HPC or hyprolose), hydroxyethylcellulose (HEC), hydroxymethylcellulose (HMC), hydroxypropyl ethylcellulose (HPEC), ethyl hydroxyethylcellulose (EHEC) and sodium carboxymethyl hydroxyethylcellulose (NaCMHEC). Examples of suitable starch derivatives include hydroxypropyl starch. In a particularly preferred embodiment, the water soluble neutral or anionic polysaccharide is a cellulose ether such as hydroxypropylcellulose.

Typically, the weight ratio of the enteric polymer to posaconazole in the granules is in the range of 5 : 1 to 1 : 1, preferably 5 : 1 to 1.5 : 1, more preferred 4 : 1 to 1 : 1, even more preferred 3.3 : 1 to 1.5 : 1, 3.1 : 1 to 1.6 : 1 or 2.8 : 1 to 1.8 : 1, and even more preferred it is 2.7 : 1 to 2.0 : 1 and finally most preferred it is 2.6 : 1 to 2.4 : 1. The granules of the present invention contain a non-enteric polymer and then, typically, the granules contain the enteric polymer and the non-enteric polymer in a weight ratio of 10 : 1 to 1 : 1, preferably of 5 : 1 to 2 : 1, and most preferred of 4 : 1 to 3 : 1.

According to a preferred embodiment of the present invention the enteric polymer is poly(methacrylic acid/ethyl acrylate) or HPMCAS and the non-enteric polymer is selected from poly(vinylpyrrolidone/vinylacetate), polyethylene glycol, hydroxypropylcellulose and polyvinylpyrrolidone, most preferred is hydroxypropylcellulose.

In a preferred embodiment of the present invention, the tablet contains an antioxidant. Preferably, the antioxidant is contained in the granules comprising posaconazole, the enteric polymer and the non-enteric polymer. Examples of antioxidants include butylated hydroxytoluene (BHT), butylated hydroxyanisole (BHA), sodium or potassium metabisulfite, sodium bisulfite, sodium sulfite, sodium thiosulfate, cysteine, acetyl cysteine, methionine, glutathione, sodium formaldehyde sulfoxylate, ascorbic acid, sodium ascorbate, ascorbyl palmitate, tocopherol, tocopheryl succinate, tocopheryl polyethylene glycol succinate (TPGS), and propyl gallate. Preferably, the antioxidant is propyl gallate. Typically, the antioxidant is present in the granules in an amount of 0.001-2 wt.-%, preferably 0.01-1 wt.-% and most preferred at about 0.4 wt.-%. Optionally, the granules of the present invention contain in addition an antioxidant synergist, e.g. citric acid, tartaric acid, or ethylenediaminetetraacetic acid (EDTA).

The granules of the present invention may additionally contain a monomeric plasticizer, e.g. triethyl citrate, triacetin, dibutyl sebacate, diethyl phthalate, glyceryl-monostearate, glycerine and propylene glycol. The monomeric plasticizer, if present, is preferably triethyl citrate and typically present in an amount of 1.0-8.0 wt.-%. preferably 3.0-6.0 wt.-%, more preferably 3.5-5 wt.-% (e.g. 4.0 wt.-%).

Posaconazole has a melting point of 170-172°C. Hence, the hot-melt extrusion used for the preparation of the gastro-resistant pharmaceutical composition of the present invention has to be conducted at temperatures of 170°C or below. Preferably, the hot-melt extrusion is conducted at a maximum temperature of 100-170°C. preferably 100-160°C, more preferred at a maximum temperature of 120-150°C and most preferred at a maximum temperature of 130-150°C such as for example 140°C. The hot-melt extrusion has to be carried out at a maximum temperature that allows the dissolution of the posaconazole used as starting material within the mixture of the enteric polymer and the non-enteric polymer. In principle, any crystalline form of posaconazole as well as the amorphous form may be used for the preparation of the gastro-resistant tablet of the present invention.

During hot melt extrusion, rotation speed of the screw is typically set in the range of 100 to 1000 rpm. According to a preferred embodiment of the present invention, the rotation speed of the screw during hot melt extrusion is in the range of 150 rpm to 300 rpm, preferably 200 rpm to 265 rpm, more preferred 225 rpm to 250 rpm. For the 11 HME Twin-screw hot melt extruder, a rotation speed of the screw of 150 to 250 rpm is particularly preferred, while for the 24 HME Twin-screw hot melt extruder, a rotation speed of the screw of 200 rpm to 265 rpm, preferably 205 to 225 rpm is particularly preferred. The rotation speed of the screw can influence, e.g., the processability during the extrusion.

The temperature of the hot-melt extrusion can be decreased when using a mixture of an enteric polymer and a non-enteric polymer, so that it is possible to process polymers with relatively high glass transitions temperatures. In addition, the non-enteric polymer, in particular the water soluble neutral or anionic polysaccharides inhibit recrystallization of posaconazole in the intestinal fluid. They act, therefore, as crystallization or precipitation inhibitors. Precipitation inhibitors are compounds capable to stabilize the supersaturation stage of the drug, i.e. they are able to prevent nucleation of the drug molecules or the growing of the initially formed drug particles, which is achieved by covering the surface of the drug particles, thereby preventing particle-particle interaction, or by enhancing the viscosity of the suspension medium. The ability of precipitation inhibitors to kinetically stabilize the supersaturated state of the drug is thought to result from intermolecular interactions between the drug and polymer in solution (e.g. via hydrogen bonding or hydrophobic interactions), the ability of the polymer to sterically hinder the crystallization process or from increasing the viscosity of the suspension medium, and not by enhancing the solubility of the drug. i.e. by increasing the equilibrium solubility (Journal of Drug Targeting 2010, 18(10), 704-731; Adv. Polym. Sci. 1993, 107, 199-265, 244). Hence, neutral or anionic polysaccharides, which are suitable for the composition of the present invention, are typically those used as suspending or thickening agents in pharmaceutical formulations.

Moreover, since the hot-melt extrusion works at relatively low temperatures, it is possible to use relatively volatile antioxidants as BHA and BHT as well as antioxidants, which degrade at processing temperatures above 140°C, e.g. sodium metabisulfite.

It has been found that the presence of a sugar alcohol in the mixture that is subjected to hot-melt extrusion may increase the chemical stability of posaconazole and also the processability, in particular, if an acidic polymer as poly(methacrylic acid/ethyl acrylate) or HPMCAS is present. Preferred sugar alcohols are xylitol, sorbitol, mannitol, maltitol, isomalt, lactitol and erythritol. The sugar alcohol, if present is preferably xylitol and typically present in the granules in an amount of 0.5-9.0 wt.-%. preferably 2.0-6.0 wt.-%

According to a preferred embodiment of the present invention, the granules consist of posaconazole, an enteric polymer, a non-enteric polymer, a monomeric plasticizer, a sugar alcohol, and optionally an antioxidant.

The granules typically comprise 10.0-35.0 wt.-% of posaconazole, 35.0-75.0 wt.-% of enteric polymer, and optionally 5.0-20.0 wt.-% of a non-enteric polymer. 1.0-8.0 wt.-% of a monomeric plasticizer, 0.5-9.0 wt.-% of a sugar alcohol and 0.001-2.0 wt-% of an antioxidant; preferably 15.0-25.0 wt.-% of posaconazole, 45.0-60.0 wt.-% of enteric polymer, 12.0-18.0 wt.-% of a non-enteric polymer, and optionally 3.0-6.0 wt.-% of a monomeric plasticizer, 2.0-6.0 wt.-% of a sugar alcohol and 0.01-1.0 wt.-% of an antioxidant.

The granules may be compressed into a tablet. Preferably the tablet contains 100 mg or 300 mg posaconazole, The tablets may be coated with a film-coating in order to make swallowing of the tablet easier. Suitable film-coating systems are commercially available under the tradename Opadry^{®}.

In a preferred embodiment, the gastro-resistant, optionally film-coated tablet of the present invention is prepared from granules containing posaconazole molecularly dispersed in a mixture containing poly(methacrylic acid/ethyl acrylate), triethyl citrate, hydroxypropylcellulose, xylitol and propyl gallate. Alternatively, posaconazole is molecularly dispersed in HPMCAS or in a mixture of HPMCAS and hydroxypropylcellulose.

The gastro-resistant, optionally film-coated tablet of the present invention may contain additional pharmaceutical excipients as extragranular component, e.g. diluents, binders, disintegrants, glidants and lubricants. Examples of diluents include microcrystalline cellulose, calcium hydrogen phosphate, lactose (anhydrous or monohydrate), and calcium carbonate. As binders may be used methyl cellulose, hydroxypropyl methylcellulose (HPMC), hydroxypropylcellulose (HPC), pregelatinized starch, povidone and copovidone. Examples of disintegrants include croscarmellose sodium, sodium starch glycolate, polyvinylpolypyrrolidone (crospovidone) and low-substituted hydroxypropylcellulose (L-HPC). As glidants silicon dioxide, talk and the like may be used, while magnesium stearate, calcium stearate, stearic acid, sodium stearyl fumarate and glycerol dibehenate are examples of suitable lubricants.

The gastro-resistant, optionally film-coated tablet of the present invention is prepared from a granulate formulation containing the granules of the present invention and pharmaceutical excipients. The preparation of the granules comprises the steps:
i) preparing a mixture containing posaconazole, the enteric polymer and optionally the non-enteric polymer, wherein the weight ratio of the enteric polymer to posaconazole is in the range of 5 : 1 to 1 : 1,
ii) subjecting the mixture obtained in step (i) to hot-melt extrusion at a temperature of 100-170°C, preferably 100-160°C, more preferably 120-150°C,
iii) milling the extrudate obtained in step (ii) to the extent that at least 10 wt.-%, preferably at least 20 wt.-%, more preferred at least 30 wt.-%, and even more preferred at least 40 wt.-% of the granules, but not more than 80 wt.-%, preferably not more than 70 wt.-%, more preferred not more than 60 wt.-% and even more preferred not more than 55 wt.-%, have a particle size of 250 µm or more, as determined by sieve analysis, wherein the milling temperature is not higher than 50°C. preferably not higher than 40°C.

Preferred embodiments of the process for preparing the granules are described in dependent claims.

The skilled person knows what are appropriate spindle speeds in the milling step, which also depends on the type of mill used. For example, when using a conical mill, the spindle speed preferably is in the range of 1.000 to 6.500 rpm. Hammer mills typically also allow a spindle speed of more than 10.000 rpm.

It was found that both the dissolution of the drug in the buffer stage is slowed down and impurities are generated if the milling temperature is set above 50°C.

The following examples are intended to further illustrate the present invention.

### Examples

Hot melt extrusion (HME) was performed with a Pharma 11 HME Twin-screw extruder (at a rotation speed of the screw of 200 +/- 50 rpm) or a Pharma 24 HME Twin-screw extruder (Examples 1-9 and Comparative Examples 1-3: at a rotation speed of the screw of 215 +/- 10 rpm: Examples 10-13: at a rotation speed of the screw of 245 +/- 20 rpm) from Thermo Fisher Scientific Inc.

In the (Comparative) Examples, in each case a Quadro conical mill, in each case model U5 or U20, has been used, if not stated otherwise. In all (Comparative) Examples, the milling of extrudes has been done with sufficient cooling so that the milling temperature is not higher than 50°C. Only in Comparative Example 2, cooling during milling was not sufficient, so that milling has taken place at a higher temperature of about 55°C.

The used film coating system Opadry^{®} II 85F520152 yellow comprises polyvinyl alcohol, titanium dioxide, polyethylene glycol/macrogol, talc and yellow iron oxide.

**In the (Comparative) Examples, the following quantitative composition has been used:**

| **Ingredients** | |
|---|---|
| | **mg** |
| **Stage-A: (Granulation)** | |
| Methacrylic acid/ethyl acrylate copolymer (1:1), Type B (Kollicoat^{®} MAE 100P) | 250 |
| Triethyl citrate | 20 |
| ***Total weight after granulation*** | **270** |

| **Stage-B: (Hot-melt extrusion, HME)** | |
|---|---|
| Granules | 270 |
| Posaconazole (Form 1) | 100 |
| Hydroxypropyl cellulose (Klucel EXF Pharma) | 75 |
| Xylitol (Xylisorb^{®} 90) | 28 |
| Propyl gallate | 2 |
| ***Total weight after HME*** | **475** |

| **Stage-C: (Blending)** | |
|---|---|
| Posaconazole HME granules | 475 |
| Hydroxypropyl cellulose (Klucel EXF Pharma) | 30 |
| Microcrystalline cellulose | 63 |
| Colloidal silicon dioxide (Aerosil^{®} 200 pharma) | 3 |
| Croscarmellose sodium | 25 |

| **Stage-D: (Lubrication)** | |
|---|---|
| Sodium stearyl fumarate (Pruv^{®}) | 4 |
| ***Core tablet weight*** | **600** |

| **Stage-E: (Film Coating)** | |
|---|---|
| Opadry^{®} II 85F520152 yellow | 24 |
| Water, purified | q.s. |
| ***Coated tablet weight*** | **624** |

In the present application, the amount of particles having a certain particle size given in the tables is in "wt.-%".

### General procedure for the preparation of the tablets:

The excipients of stage A were sifted and granulated. Posaconazole and the excipients of stage B were sifted and blended with the granules of stage A. The mixture was subjected to hot-melt extrusion and the obtained extrudate was milled thus resulting in milled granules as claimed according to the present invention. Microcrystalline cellulose, hydroxypropylcellulose, silicon dioxide, croscarmellose sodium and sodium stearyl fumarate were sifted and blended with the milled extrudate (milled granules) and then subjected to compression to obtain a tablet, which was finally film-coated with Opadry^{®} II Yellow.

The dissolution tests were performed according to Monograph '2.9.3 Dissolution of the European Pharmacopeia 10.0 with the following conditions: USP apparatus II (paddle); speed: 75 rpm; acid stage: 750 ml of 0.01 N HCl) for 2 h, followed by buffer stage: pH 6.8 phosphate buffer containing 0.365 % polysorbate 80 (1000 ml); time points: acid stage: 120 min, and buffer stage: 5, 10, 15. 20. 30. 45and 60 min; samples: 6 units.

### Example 1

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol AI) | 20 |
| ***Weight of Granules*** | **270** |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including milling):

| **Parameters (Zones and Temperatures)** | **Temperature °C** |
|---|---|
| Zone 2 | 40 |
| Zone 3 | 80 |
| Zone 4 | 120 |
| Zone 5 | 140 |
| Zone 6 | 140 |
| Zone 7 | 140 |
| Zone 8 | 140 |
| Die | 140 |
| Screens | 32G (813µm) →24R (610µm) →18R (457µm) |
| Milling rpm | 4000 |
| #60 (250µm) | 50.00 |
| #80 (180µm) | 53.33 |
| #100 (150µm) | 76.67 |
| #120 (125µm) | 83.33 |
| #170 (90µm) | 90.00 |
| Receiver | 100.00 |

| **Parameters of the core tablets** | |
|---|---|
| Tablet weight (mg) | 596-603 |
| Thickness (mm) | 6.38-6.40 |
| Hardness (N) | 122-141 |
| Disintegration time (min) | 13'20" |

| **Parameters of the film-coated tablets** | |
|---|---|
| Weight (mg) | 620-629 |
| Thickness (mm) | 6.49-6.58 |
| Hardness (N) | 165-182 |
| D.T. (min) | 16'45" |

| **Time [min]** | **Dissolution / Drug Release [%] in 0.01 N HCl for 2 h followed by pH 6.8 phosphate buffer** |
|---|---|
| **Acid stage** | |
| 120 | 5 |

| **Buffer stage** | |
|---|---|
| 5 | 82 |
| 10 | 95 |
| 15 | 101 |
| 20 | 102 |
| 30 | 102 |
| 45 | 102 |
| 60 | 102 |

### Example 2

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including milling):

| **Parameters (Zones and Temperatures)** | **Temperature °C** |
|---|---|
| Zone 2 | 40 |
| Zone 3 | 80 |
| Zone 4 | 120 |
| Zone 5 | 140 |
| Zone 6 | 140 |
| Zone 7 | 140 |
| Zone 8 | 140 |
| Die | 140 |
| Screens | Only 32G (813 µm) Screen |
| Milling rpm | 4000 |
| #60 (250µm) | 70.00 |
| #80 (180µm) | 73.33 |
| #100 (150µm) | |
| #120 (125µm) | 83.33 86.67 |
| #170 (90µm) | 93.33 |
| Receiver | 100.00 |

| **Parameters of the core tablets** | |
|---|---|
| Tablet weight (mg) | 598-606 |
| Thickness (mm) | 6.29-6.36 |
| Hardness (N) | 128-141 |
| Disintegration time (min) | 13'45" |

| **Parameters of the film-coated tablets** | |
|---|---|
| Tablet weight (mg) | 620-628 |
| Thickness (mm) | 6.48-6.56 |
| Hardness (N) | 159-170 |
| Disintegration time (min) | 14'45" |

| **Time [min]** | **Dissolution / Drug Release [%] in 0.01 N HCl for 2 h followed by pH 6.8 phosphate buffer** |
|---|---|
| **Acid stage** | |
| 120 | 5 |

| **Buffer stage** | |
|---|---|
| 5 | 51 |
| 10 | 79 |
| 15 | 92 |
| 20 | 96 |
| 30 | 99 |
| 45 | 99 |
| 60 | 99 |

### Example 3

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including milling):

| **Parameters (Zones and Temperatures)** | **Temperature °C** |
|---|---|
| Zone 2 | 40 |
| Zone 3 | 80 |
| Zone 4 | 120 |
| Zone 5 | 140 |
| Zone 6 | 140 |
| Zone 7 | 140 |
| Zone 8 | 140 |
| Die | 140 |
| Screens | 32G (813µm) →24R (610µm) |
| Milling rpm | 4000 |
| #60 (250µm) | 55.17 |
| #80 (180µm) | 62.07 |
| #100 (150µm) | 68.97 |
| #120 (125µm) | 72.41 |
| #170 (90µm) | 86.21 |
| Receiver | 100.00 |

| **Parameters of the core tablets** | |
|---|---|
| Tablet weight (mg) | 598-605 |
| Thickness (mm) | 6.31-6.39 |
| Hardness (N) | 129-139 |
| Disintegration time (min) | 13'20" |

| **Parameters of the film-coated tablets** | |
|---|---|
| Tablet weight (mg) | 620-629 |
| Thickness (mm) | 6.48-6.57 |
| Hardness (N) | 158-170 |
| Disintegration time (min) | 14'30" |

| **Time [min]** | **Dissolution / Drug Release [%] in 0.01 N HCl for 2 h followed by pH 6.8 phosphate buffer** |
|---|---|
| **Acid stage** | |
| 120 | 6 |

| **Buffer stage** | |
|---|---|
| 5 | 56 |
| 10 | 86 |
| 15 | 98 |
| 20 | 102 |
| 30 | 103 |
| 45 | 103 |
| 60 | 103 |

### Comparative Example 1

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including milling):

| **Parameters (Zones and Temperatures)** | **Temperature °C** |
|---|---|
| Zone 2 | 40 |
| Zone 3 | 80 |
| Zone 4 | 120 |
| Zone 5 | 140 |
| Zone 6 | 140 |
| Zone 7 | 140 |
| Zone 8 | 140 |
| Die | 140 |
| Screens | 62G (1575 µ) |
| Milling rpm | 4000 |
| #60 (250µm) | 87.5 |
| #80 (180µm) | 90.63 |
| #100 (150µm) | 90.63 |
| #120 (125µm) | 93.75 |
| #170 (90µm) | 96.88 |
| Receiver | 100.00 |

| **Parameters of the core tablets** | |
|---|---|
| Tablet weight (mg) | 596-606 |
| Thickness (mm) | 6.23-6.32 |
| Hardness (N) | 98-105 |
| Disintegration time (min) | 13'09" |

| **Parameters of the film-coated tablets** | |
|---|---|
| Tablet weight (mg) | 620-629 |
| Thickness (mm) | 6.48-6.54 |
| Hardness (N) | 138-148 |
| Disintegration time (min) | 15'10" |

| **Time [min]** | **Dissolution / Drug Release [%] in 0.01 N HCl for 2 h followed by pH 6.8 phosphate buffer** |
|---|---|
| **Acid stage** | |
| 120 | 2 |

| **Buffer stage** | |
|---|---|
| 5 | 34 |
| 10 | 55 |
| 15 | 70 |
| 20 | 79 |
| 30 | 87 |
| 45 | 92 |
| 60 | 93 |

Drug dissolution was too slow in the buffer stage.

### Example 4

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including milling):

| **Parameters (Zones and Temperatures)** | **Temperature °C** |
|---|---|
| Zone 2 | 40 |
| Zone 3 | 80 |
| Zone 4 | 120 |
| Zone 5 | 140 |
| Zone 6 | 140 |
| Zone 7 | 140 |
| Zone 8 | 140 |
| Die | 140 |
| Screens | 32G (813µm) → 24R (610µm) → 18R (457µm) → 11R (279 µm) |
| Milling rpm | 4000 |
| #60 (250µm) | 24.14 |
| #80 (180µm) | 44.83 |
| #100 (150µm) | 58.62 |
| #120 (125µm) | 68.97 |
| #170 (90µm) | 82.76 |
| Receiver | 100.00 |

| **Parameters of the core tablets** | |
|---|---|
| Tablet weight (mg) | 598-604 |
| Thickness (mm) | 6.40-6.46 |
| Hardness (N) | 128-140 |
| Disintegration time (min) | 11'40" |

| **Parameters of the film-coated tablets** | |
|---|---|
| Tablet weight (mg) | 620-629 |
| Thickness (mm) | 6.48-6.53 |
| Hardness (N) | 159-170 |
| Disintegration time (min) | 13'35" |

| **Time [min]** | **Dissolution / Drug Release [%] in 0.01 N HCl for 2 h followed by pH 6.8 phosphate buffer** |
|---|---|
| **Acid stage** | |
| 120 | 8 |

| **Buffer stage** | |
|---|---|
| 5 | 81 |
| 10 | 98 |
| 15 | 101 |
| 20 | 101 |
| 30 | 101 |
| 45 | 101 |
| 60 | 101 |

### Example 5

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | 475 |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including millng):

| **Parameters (Zones and Temperatures)** | **Temperature °C** |
|---|---|
| Zone 2 | 40 |
| Zone 3 | 80 |
| Zone 4 | 120 |
| Zone 5 | 140 |
| Zone 6 | 140 |
| Zone 7 | 140 |
| Zone 8 | 140 |
| Die | 140 |

| Screens | 32G (813µm) → 24R (610µm) → 18R (457µm) → 11R (279 µm) →9R (229µm) |
|---|---|
| Milling rpm | 4000 |
| #60 (250µm) | 9.68 |
| #80 (180µm) | 12.9 |
| #100 (150µm) | 41.94 |
| #120 (125µm) | 58.06 |
| #170 (90µm) | 70.97 |
| Receiver | 100.00 |

| **Parameters of the core tablets** | |
|---|---|
| Tablet weight (mg) | 600-609 |
| Thickness (mm) | 6.41-6.51 |
| Hardness (N) | 125-145 |
| Disintegration time (min) | 5'55" |

| **Parameters of the film-coated tablets** | |
|---|---|
| Tablet weight (mg) | 622-628 |
| Thickness (mm) | 6.61-6.72 |
| Hardness (N) | 146-181 |
| Disintegration time (min) | 6'15" |

| **Time [min]** | **Dissolution / Drug Release [%] in 0.01 N HCl for 2 h followed by pH 6.8 phosphate buffer** |
|---|---|
| **Acid stage** | |
| 120 | 10 |

| **Buffer stage** | |
|---|---|
| 5 | 89 |
| 10 | 97 |
| 15 | 99 |
| 20 | 99 |
| 30 | 100 |
| 45 | 99 |
| 60 | 99 |

Examples 1 to 5, and Comparative Example 1 show that the dissolution rate of the drug increases with the particle size of the granules, both in the acid as well as in the buffer stage.

### Example 6

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol Al) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxy propylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |

| | |
|---|---|
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (milling):

| **Parameters (Zones and Temperatures)** | **Temperature °C** |
|---|---|
| Zone 2 | 40 |
| Zone 3 | 80 |
| Zone 4 | 120 |
| Zone 5 | 120 |
| Zone 6 | 120 |
| Zone 7 | 120 |
| Zone 8 | 120 |
| Die | 120 |
| Screens | 32G (813µm) → 24R (610µm) → 18R (457µm) |
| Milling rpm | 4000 |
| #60 (250µm) | 51.72 |
| #80 (180µm) | 55.17 |
| #100 (150µm) | 72.41 |
| #120 (125µm) | 79.31 |
| #170 (90µm) | 86.21 |
| Receiver | 100.00 |

| **Parameters of the core tablets** | |
|---|---|
| Tablet weight (mg) | 598-606 |
| Thickness (mm) | 6.45-6.49 |
| Hardness (N) | 130-143 |
| Disintegration time (min) | 12'20" |

| **Parameters of the film-coated tablets** | |
|---|---|
| Tablet weight (mg) | 620-626 |
| Thickness (mm) | 6.49-6.59 |
| Hardness (N) | 159-180 |
| Disintegration time (min) | 14'45" |

| **Time [min]** | **Dissolution / Drug Release [%] in 0.01 N HCl for 2 h followed by pH 6.8 phosphate buffer** |
|---|---|
| **Acid stage** | |
| 120 | 5 |

| **Buffer stage** | |
|---|---|
| 5 | 87 |
| 10 | 99 |
| 15 | 103 |
| 20 | 104 |
| 30 | 104 |
| 45 | 104 |
| 60 | 104 |

### Example 7

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including milling):

| **Parameters (Zones and Temperatures)** | **Temperature °C** |
|---|---|
| Zone 2 | 40 |
| Zone 3 | 80 |
| Zone 4 | 120 |
| Zone 5 | 160 |
| Zone 6 | 160 |
| Zone 7 | 160 |
| Zone 8 | 160 |
| Die | 160 |
| Screens | 32G (813µm) → 24R (610µm) → 18R (457µm) |
| Milling rpm | 4000 |
| #60 (250µm) | 51.61 |
| #80 (180µm) | 58.06 |
| #100 (150µm) | 74.19 |
| #120 (125µm) | 80.65 |
| #170 (90µm) | 83.87 |
| Receiver | 100.00 |

| **Parameters of the core tablets** | |
|---|---|
| Tablet weight (mg) | 598-605 |
| Thickness (mm) | 6.44-6.48 |
| Hardness (N) | 125-140 |
| Disintegration time (min) | 14'10" |

| **Parameters of the film-coated tablets** | |
|---|---|
| Tablet *weight* (mg) | 621-629 |
| Thickness (mm) | 6.50-6.59 |
| Hardness (N) | 166-181 |
| Disintegration time (min) | 16'20" |

| **Time [min]** | **Dissolution / Drug Release [%]** in **0.01 N HCl for 2 h followed by pH 6.8 phosphate buffer** |
|---|---|
| **Acid stage** | |
| 120 | 5 |

| **Buffer stage** | |
|---|---|
| 5 | 69 |
| 10 | 94 |
| 15 | 102 |
| 20 | 103 |
| 30 | 103 |
| 45 | 103 |
| 60 | 103 |

### Example 8

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | 475 |

| **Stage-C: (Lubrication)** | |
|---|---|
| Microcrystalline Cellulose (Comprecel M 102D+) | 63 |
| Hydroxyoropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including milling):

| **Parameters (Zones and Temperatures)** | **Temperature °C** |
|---|---|
| Zone 2 | 40 |
| Zone 3 | 80 |
| Zone 4 | 120 |
| Zone 5 | 140 |
| Zone 6 | 140 |
| Zone 7 | 140 |
| Zone 8 | 140 |
| Die | 140 |

| | |
|---|---|
| Screens | 32G (813µm) → 24R (610µm) → 18R (457µm) |
| Milling rpm | 1000 |
| #60 (250um) | 62.50 |
| #80 (180µm) | 71.88 |
| #100 (150µm) | 81.25 |
| #120 (125µm) | 87.50 |
| #170 (90µm) | 93.75 |
| Receiver | 100.00 |

| **Parameters of the core tablets** | |
|---|---|
| Tablet weight (mg) | 596-608 |
| Thickness (mm) | 6.36-6.43 |
| Hardness (N) | 125-136 |
| Disintegration time (min) | 13'25" |

| **Parameters of the film-coated tablets** | |
|---|---|
| Tablet weight (mg) | 620-629 |
| Thickness (mm) | 6.49-6.60 |
| Hardness (N) | 165-175 |
| Disintegration time (min) | 14'20" |

| **Time [min]** | **Dissolution / Drug Release [%] in 0.01 N HCl for 2 h followed by pH 6.8 phosphate buffer** |
|---|---|
| **Acid stage** | |
| 120 | 5 |

| **Buffer stage** | |
|---|---|
| 5 | 72 |
| 10 | 92 |
| 15 | 98 |
| 20 | 100 |
| 30 | 100 |
| 45 | 100 |
| 60 | 100 |

### Example 9

| **Ingredients** | **mg** |
|---|---|
| **Stage-A: (Granulation)** | |
| Methacrylic Acid/Ethyl Acrylate Copolymer (1:1). Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including milling):

| **Parameters (Zones and Temperatures)** | **Temperature °C** |
|---|---|
| Zone 2 | 40 |
| Zone 3 | 80 |
| Zone 4 | 120 |
| Zone 5 | 140 |
| Zone 6 | 140 |
| Zone 7 | 140 |
| Zone 8 | 140 |
| Die | 140 |
| Screens | 32G (813µm) → 24R (610µm) → 18R (457µm) |
| Milling rpm | 6500 |
| #60 (250µm) | 42.86 |
| #80 (180µm) | 60.71 |
| #1 00 (150µm) | 71.43 |
| #120 (125µm) | 78.57 |
| *#*170 (90µm) | 89.29 |
| Receiver | 100.00 |

| **Parameters of the core tablets** | |
|---|---|
| Tablet weight (mg) | 598-605 |
| Thickness (mm) | 6.31-6.39 |
| Hardness (N) | 125-140 |
| Disintegration time (min) | 13'46" |

| **Parameters of the film-coated tablets** | |
|---|---|
| Tablet weight (mg) | 620-629 |
| Thickness (mm) | 6.48-6.52 |
| Hardness (N) | 161-179 |
| Disintegration time (min) | 13'35" |

| **Time [min]** | **Dissolution** / **Drug Release [%] in 0.01 N HCl for 2 h followed by pH 6.8 phosphate buffer** |
|---|---|
| **Acid stage** | |
| 120 | 6 |

| **Buffer stage** | |
|---|---|
| 5 | 82 |
| 10 | 98 |
| 15 | 101 |
| 20 | 102 |
| 30 | 101 |
| 45 | 102 |
| 60 | 101 |

### Comparative Example 2

| **Ingredients** | **mg** |
|---|---|
| **Stage-A: (Granulation)** | |
| Methacrylic Acid/Ethyl Acrylate Copolymer (1:1) Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadryl^{®} II Yellow 85F520152 | 24 |
| Water, Purified | q.s. |
| ***Film-coated tablet weight*** | 624 |

### HME process (including milling):

| **Parameters (Zones and Temperatures)** | **Temperature °C** |
|---|---|
| Zone 2 | 40 |
| Zone 3 | 80 |
| Zone 4 | 120 |
| Zone 5 | 140 |
| Zone 6 | 140 |
| Zone 7 | 140 |
| Zone 8 | 140 |
| Die | 140 |
| Screens | 32G (813µm) → 24R (610µm) → 18R (457µm) (U20 mill) |
| Milling rpm | 1640 |
| #60 (250µm) | 57.3 |
| #80 (180µm) | 70.42 |
| #100 (150µm) | 76.74 |
| #120 (125µm) | 78.62 |
| #170 (90µm) | 89.10 |
| Receiver | 100.00 |

| **Parameters of the core tablets** | |
|---|---|
| Tablet weight (mg) | 584-625 |
| Thickness (mm) | 6.08-6.26 |
| Hardness (N) | 98-137 |
| Disintegration time (min) | 23'30" |

| **Parameters of the film-coated tablets** | |
|---|---|
| Tablet weight (mg) | 625 |
| Thickness (mm) | 6.25-6.45 |
| Hardness (N) | 135-168 |
| Disintegration time (min) | 32'10" |

| **Time [min]** | **Dissolution** / **Drug Release [%] in 0.01 N HCl for 2 h followed by pH 6.8 phosphate buffer** |
|---|---|
| **Acid stage** | |
| 120 | 6 |

| **Buffer stage** | |
|---|---|
| 5 | - |
| 10 | 77 |
| 15 | 83 |
| 20 | 86 |
| 30 | 88 |
| 45 | 86 |
| 60 | - |

It was found that both the dissolution of the drug in the buffer stage is slowed down and impurities are generated, because the milling temperature was too high (55 °C).

### Comparative Example 3

| **Ingredients** | **mg** |
|---|---|
| **Stage-A: (Granulation)** | |
| Methacrylic Acid/Ethyl Acrylate Copolymer (1:1), Type B (Kollicoat MAE 100P) | 250 |
| Triethyl Citrate (Citrifol AI) | 20 |
| ***Weight of Granules*** | 270 |

| **Stage-B: (Hot-melt extrusion)** | |
|---|---|
| Posaconazole (Form-1) | 100 |
| Xylitol (Xylisorb 90) | 28 |
| Hydroxypropylcellulose (Klucel EXF) | 75 |
| Propyl Gallate | 2 |
| ***Weight of HME Granules*** | **475** |

| **Stage-C: (Lubrication)** | |
|---|---|
| Microcrystalline Cellulose (Comprecel M 102D+) | 63 |
| Hydroxypropylcellulose (Klucel EXF) | 30 |
| Colloidal silicon dioxide (Aerosil 200 pharma) | 3 |
| Croscarmellose Sodium (Ac-Di-Sol) | 25 |
| Sodium Stearyl Fumarate | 4 |
| ***Core tablet weight*** | **600** |

| **Stage-D: (Film Coating)** | |
|---|---|
| Opadry^{®} II Yellow 85F520152 | 24 |
| Water, Purified | q.s. |
| ***Film-coated tablet weight*** | **624** |

### HME process (including milling):

| **Parameters (Zones and Temperatures)** | **Temperature °C** |
|---|---|
| Zone 2 | 40 |
| Zone 3 | 80 |
| Zone 4 | 90 |
| Zone 5 | 90 |
| Zone 6 | 90 |
| Zone 7 | 90 |
| Zone 8 | 90 |
| Die | 90 |

| | |
|---|---|
| Screens | 32G (813µm) → 24R (610µm) → 18R (457µm) |
| Milling rpm | 4000 |
| #60 (250µm) | 53.33 |
| #80 (180µm) | 56.67 |
| #100 (150µm) | 76.67 |
| #120 (125µm) | 80.00 |
| #170 (90µm) | 86.67 |
| Receiver | 100.00 |

| **Parameters of the core tablets** | |
|---|---|
| Tablet weight (mg) | 596-605 |
| Thickness (mm) | 6.27-6.35 |
| Hardness (N) | 125-135 |
| Disintegration time (min) | 26'15" |

| **Parameters of the film-coated tablets** | |
|---|---|
| Tablet weight (mg) | 620-628 |
| Thickness (mm) | 6.47-6.52 |
| Hardness (N) | 166-180 |
| Disintegration time (min) | 27'29". |

| **Time [min]** | **Dissolution / Drug Release [%] in 0.01 N HCl for 2 h followed by pH 6.8 phosphate buffer** |
|---|---|
| **Acid stage** | |
| 120 | 4 |

| **Buffer stage** | |
|---|---|
| 5 | 57 |
| 10 | 79 |
| 15 | 89 |
| 20 | 93 |
| 30 | 98 |
| 45 | 101 |
| 60 | 102 |

The dissolution requirements were met at the low HME temperature, wherein dissolution of the drug is slowed down in both the acid stage and the buffer stage compared to higher HME temperatures (see the dissolution profiles of Examples 6, and 7). Also, the low HME temperature increased torque to such an extent that the extruder stopped.

### Examples 10-13

| **Ingredients** | **Ex. 10 [mg]** | **Ex. 11 [mg]** | **Ex. 12 [mg]** | **Ex. 13 [mg]** |
|---|---|---|---|---|
| **Stage-A (Blending Hot-melt extrusion)** | | | | |
| Posaconazole (Form-1) | 300 | 300 | 300 | 300 |
| Hypromellose Acetate Succinate | 975 | 975 | 900 | 900 |
| Hydroxypropylcellulose | - | - | 75 | 75 |
| ***Weight of HME Granules*** | **1275** | **1275** | **1275** | **1275** |

| **Stage-B (Lubrication)** | | | | |
|---|---|---|---|---|
| Cellulose, Microcrystalline | 50 | 50 | 50 | 50 |
| Hydroxypropylcellulose | 50 | 50 | 50 | 50 |
| Silica, Colloidal Anhydrous | 5 | 5 | 5 | 5 |
| Croscarmellose Sodium | 45 | 45 | 45 | 45 |
| Sodium Stearyl Fumarate | 5 | 5 | 5 | 5 |
| ***Core tablet weight*** | **1430** | **1430** | **1430** | **1430** |

| **Stage-C (Film Coating)** | | | | |
|---|---|---|---|---|
| Opadry^{®} II Yellow 85F520152 | 35 | 35 | 35 | 35 |
| Water, Purified | q.s. | q.s. | q.s. | q.s. |
| ***Coated tablet weight*** | **1465** | **1465** | **1465** | **1465** |

| **Stage-D (Clear Coating)** | | | | |
|---|---|---|---|---|
| Opadry^{®} EZ Easy Swallow Clear 253U190007 | 15 | 15 | 15 | 15 |
| Water, Purified | q.s. | q.s. | q.s. | q.s. |
| ***Coated tablet weight*** | **1480** | **1480** | **1480** | **1480** |

### Procedure for the preparation of the tablets:

The excipients of stage A were sifted and mixed. The mixture was subjected to hot-melt extrusion and the obtained extrudate was milled thus resulting in milled granules as claimed according to the present invention. Microcrystalline cellulose, hydroxypropylcellulose, silicon dioxide, croscarmellose sodium and sodium stearyl fumarate were sifted and blended with the milled extrudate (milled granules) and then subjected to compression to obtain a tablet, which was finally film-coated with Opadry^{®} II Yellow and Opadry^{®} EZ Easy Swallow Clear.

### HME process (including milling):

| **Parameters (Zones)** | **Temperature °C** |
|---|---|
| Zone 2 | 40 ± 20 |
| Zone 3 | 80 ± 10 |
| Zone 4 | 120 ± 10 |
| Zone 5 | 160 ± 10 |
| Zone 6 | 160 ± 10 |
| Zone 7 | 160 ± 10 |
| Zone 8 | 160 ± 10 |
| Die | 160 ± 10 |
| Screens (Hammer Mill) | Ex. 10 and 12: 1.0 mm |
| | Ex. 11 and 13: 1.0 mm → 0.5 mm |
| Milling rpm | 12000 rpm |

### Particle size distribution of milled extrudes by sieve analysis:

| **Sieve No (Microns)** | **Cumulative % Retained** | | | |
|---|---|---|---|---|
| | **Ex. 10** | **Ex. 11** | **Ex. 12** | **Ex. 13** |
| #60 (250 µm) | 36.00 | 16.00 | 36.73 | 17.65 |
| 100 (150 µm) | 68.00 | 62.00 | 69.39 | 62.75 |
| #120 (125 µm) | 76.00 | 72.00 | 75.51 | 70.59 |
| #170 (90 µm) | 86.00 | 82.00 | 83.67 | 80.39 |
| Receiver | 100.00 | 100.00 | 100.00 | 100.00 |

### Physiochemical parameters of core and coated tablets:

| **Parameters of the core tablets** | **Ex. 10** | **Ex. 11** | **Ex. 12** | **Ex. 13** |
|---|---|---|---|---|
| Mass (mg) | 1434.3 | 1435.9 | 1435.6 | 14.36.5 |
| Thickness (mm) | 8.23 | 8.38 | 8.32 | 8.44 |
| Hardness (N) | 202 | 204 | 210 | 200 |
| Disintegration time | 2 min 20 sec | 1 min 30 sec | 5 min 30 sec | 5 min 10 sec |

| **Parameters of the coated tablets** | | | | |
|---|---|---|---|---|
| Mass (mg) | 1482.2 | 1480 | 1482 | 1480 |
| Thickness (mm) | 8.41 | 8.47 | 8.41 | 8.55 |
| Hardness (N) | 249 | 251 | 251 | 241 |
| Disintegration time | 2 min 45 sec | 1 min 45 sec | 5 min 20 sec | 5 min 25 sec |

### Dissolution profiles in 0.01 N HCl, 750 ml, paddle, 75 rpm followed by phosphate buffer pH 6.8 with 1.1% Polysorbate 80, make up to 1000 ml. paddle, 75 rpm (Release media):

| | **Cumulative % Labelled Amount Dissolved** | | | | | | | |
|---|---|---|---|---|---|---|---|---|
| | **Acid stage** | **Buffer stage** | | | | | | |
| | **120 min** | **5 min** | **10 min** | **15 min** | **20 min** | **30 min** | **45 min** | **60 min** |
| 3×Noxafil 100 mg | 4 | 77 | 93 | 98 | 99 | 100 | 100 | 100 |
| Ex. 10 | 3 | 69 | 82 | 88 | 91 | 93 | 94 | 95 |
| Ex. 11 | 4 | 83 | 96 | 97 | 97 | 97 | 98 | 97 |
| Ex. 12 | 6 | 74 | 88 | 94 | 96 | 96 | 96 | 96 |
| Ex. 13 | 8 | 85 | 95 | 97 | 97 | 97 | 98 | 98 |

## Claims

1. Granules comprising posaconazole molecularly dispersed in a mixture containing an enteric polymer and optionally a non-enteric polymer, wherein the weight ratio of the enteric polymer to posaconazole is in the range of 5 : 1 to 1 : 1, and wherein at least 10 wt.-% of the granules, but not more than 80 wt.-%, have a particle size of 250 µm or more, as determined by sieve analysis.

2. The granules according to claim 1, wherein at least 20 wt.-% of the granules, but not more than 70 wt.-%, have a particle size of 250 µm or more, as determined by sieve analysis.

3. The granules according to any one of the preceding claims, wherein at least 30 wt.-% of the granules, but not more than 60 wt.-%, have a particle size of 250 µm or more, as determined by sieve analysis.

4. The granules according to any one of the preceding claims, wherein at least 40 wt.-% of the granules, but not more than 55 wt.-%, have a particle size of 250 µm or more, as determined by sieve analysis.

5. The granules according to any one of the preceding claims, wherein d (0.1) of the granules is at least 60 µm, preferably at least 75 µm, and more preferred at least 100 µm, and/or is not more than 250 µm, preferably not more than 180 µm, and more preferred not more than 130 µm, as measured by dynamic light scattering, dry method.

6. The granules according to any one of the preceding claims, wherein d (0.5) of the granules is at least 160 µm, preferably at least 200 µm, and more preferred at least 250 µm, and/or is not more than 500 µm, preferably not more than 400 µm, and more preferred not more than 320 µm, as measured by dynamic light scattering, dry method.

7. The granules according to any one of the preceding claims, wherein d (0.9) of the granules is at least 300 µm, preferably at least 400 µm, and more preferred at least 480 µm, and/or is not more than 1000 µm, preferably not more than 800 µm, and more preferred not more than 600 µm, as measured by dynamic light scattering, dry method.

8. The granules according to any one of the preceding claims, wherein the enteric polymer is hypromellose acetate succinate (HPMCAS) and/or a polymethacrylic acid derivative selected from poly(methacrylic acid/methyl methacrylate) and poly(methacrylic acid/ethyl acrylate), and preferably is poly(methacrylic acid/ethyl acrylate).

9. The granules according to any one of the preceding claims, wherein the non-enteric polymer is selected from polyvinylpyrrolidone, poly(vinylpyrrolidone/ vinylacetate), polyvinylcaprolactam/polyvinylacetate/polyethylene glycol graft copolymer, polyethylene glycol/polyvinyl alcohol graft copolymer, poly(ethylene oxide/propylene oxide), macrogolglycerol hydroxystearate, polyethylene glycol and a water soluble neutral or anionic polysaccharide, and preferably is a water soluble neutral or anionic polysaccharide.

10. The granules according to claim 9, wherein the water soluble neutral or anionic polysaccharide is selected from a gum, pectin, dextran, dextrin, a cellulose ether, pregelatinized starch and a starch ether, and preferably is a cellulose ether such as hydroxypropylcellulose.

11. The granules according to any one of the preceding claims, wherein the mixture contains an antioxidant, wherein the antioxidant preferably is selected from butylated hydroxytoluene (BHT), butylated hydroxyanisole (BHA), sodium or potassium metabisulfite, sodium bisulfite, sodium sulfite, sodium thiosulfate, cysteine, acetyl cysteine, methionine, glutathione, sodium formaldehyde sulfoxylate, ascorbic acid, sodium ascorbate, ascorbyl palmitate, tocopherol, tocopheryl succinate, tocopheryl polyethylene glycol succinate (TPGS) and propyl gallate, and particularly preferred is propyl gallate.

12. The granules according to any one of the preceding claims, wherein the mixture contains a monomeric plasticizer,, wherein the monomeric plasticizer preferably is selected from triethyl citrate, triacetin, dibutyl sebacate, diethyl phthalate, glycerylmonostearate, glycerine and propylene glycol, and particularly preferred is triethyl citrate.

13. The granules according to any one of the preceding claims, wherein the mixture contains a sugar alcohol, wherein the sugar alcohol preferably is selected from xylitol, sorbitol, mannitol and maltitol, and particularly preferred is xylitol.

14. The granules according to any one of the preceding claims, wherein the granules consist of posaconazole, the enteric polymer, the non-enteric polymer, the monomeric plasticizer, the sugar alcohol, and optionally the antioxidant.

15. The granules according to any one of the preceding claims, wherein the mixture is prepared by hot-melt extrusion.

16. A process for preparing the granules according to any one of claims 1-15, comprising the steps:
i) preparing a mixture containing posaconazole, the enteric polymer and optionally the non-enteric polymer, wherein the weight ratio of the enteric polymer to posaconazole is in the range of 5 : 1 to 1 : 1,
ii) subjecting the mixture obtained in step (i) to hot-melt extrusion at a temperature of 100-170°C, preferably 100-160°C, more preferably 120-150°C,
iii) milling the extrudate obtained in step (ii) to the extent that at least 10 wt.-%, preferably at least 20 wt.-%, more preferred at least 30 wt.-%, and even more preferred at least 40 wt.-% of the granules, but not more than 80 wt.-%, preferably not more than 70 wt.-%, more preferred not more than 60 wt.-%, and even more preferred not more than 55 wt.-%, have a particle size of 250 µm or more, as determined by sieve analysis, wherein the milling temperature is not higher than 50°C, preferably not higher than 40°C.

17. A process according to claim 16, wherein the rotation speed of the screw during hot melt extrusion is in the range of 150 rpm to 300 rpm, preferably 200 rpm to 265 rpm, more preferred 225 rpm to 250 rpm.

18. A gastro-resistant, optionally film-coated tablet comprising the granules according to any one of claims 1-15 and a pharmaceutical excipient.

19. The gastro-resistant, optionally film-coated tablet according to claim 18, wherein the pharmaceutical excipient is selected from a diluent, binder, disintegrant, glidant and lubricant.

20. The gastro-resistant, optionally film-coated tablet according to claim 19, wherein posaconazole is molecularly dispersed in a mixture containing poly(methacrylic acid/ethyl acrylate), triethyl citrate, hydroxypropylcellulose, xylitol and propyl gallate.

21. The gastro-resistant, optionally film-coated tablet according to claim 19, wherein posaconazole is molecularly dispersed in HPMCAS or in a mixture of HPMCAS and hydroxypropylcellulose.

## Patentansprüche

1. Granulat, umfassend Posaconazol molekular dispergiert in einem Gemisch, das ein magensaftresistentes Polymer und gegebenenfalls ein nicht magensaftresistentes Polymer enthält, wobei das Gewichtsverhältnis des magensaftresistenten Polymers zu Posaconazol im Bereich von 5:1 bis 1:1 ist und wobei mindestens 10 Gew.-% des Granulats, aber nicht mehr als 80 Gew.-%, eine Partikelgröße von 250 µm oder mehr aufweist, bestimmt mittels Siebanalyse.

2. Granulat gemäß Anspruch 1, wobei mindestens 20 Gew.-% des Granulats, aber nicht mehr als 80 Gew.-%, eine Partikelgröße von 250 µm oder mehr aufweist, bestimmt mittels Siebanalyse.

3. Granulat gemäß einem der vorstehenden Ansprüche, wobei mindestens 30 Gew.-% des Granulats, aber nicht mehr als 60 Gew.-%, eine Partikelgröße von 250 µm oder mehr aufweist, bestimmt mittels Siebanalyse.

4. Granulat gemäß einem der vorstehenden Ansprüche, wobei mindestens 40 Gew.-% des Granulats, aber nicht mehr als 55 Gew.-%, eine Partikelgröße von 250 µm oder mehr aufweist, bestimmt mittels Siebanalyse.

5. Granulat gemäß einem der vorstehenden Ansprüche, wobei d(0,1) des Granulats mindestens 60 µm beträgt, vorzugsweise mindestens 75 µm und mehr bevorzugt mindestens 100 µm, und/oder nicht mehr als 250 µm beträgt, vorzugsweise nicht mehr als 180 µm und mehr bevorzugt nicht mehr als 130 µm, gemessen mittels dynamischer Lichtstreuung, Trockenverfahren.

6. Granulat gemäß einem der vorstehenden Ansprüche, wobei d(0,5) des Granulats mindestens 160 µm beträgt, vorzugsweise mindestens 200 µm und mehr bevorzugt mindestens 250 µm, und/oder nicht mehr als 500 µm beträgt, vorzugsweise nicht mehr als 400 µm und mehr bevorzugt nicht mehr als 320 µm, bestimmt mittels dynamischer Lichtstreuung, Trockenverfahren.

7. Granulat gemäß einem der vorstehenden Ansprüche, wobei d(0,9) des Granulats mindestens 300 µm beträgt, vorzugsweise mindestens 400 µm und mehr bevorzugt mindestens 480 µm, und/oder nicht mehr als 1000 µm beträgt, vorzugsweise nicht mehr als 800 µm und mehr bevorzugt nicht mehr als 600 µm, bestimmt mittels dynamischer Lichtstreuung, Trockenverfahren.

8. Granulat gemäß einem der vorstehenden Ansprüche, wobei das magensaftresistente Polymer ausgewählt ist aus Hypromelloseacetatsuccinat (HPMCAS) und/oder einem Polymethacrylsäure-Derivat, das aus Poly(methacrylsäure/methylmethacrylat) und Poly(methacrylsäure/ethylacrylat) ausgewählt ist, und vorzugsweise Poly(methacrylsäure/ethylacrylat) ist.

9. Granulat gemäß einem der vorstehenden Ansprüche, wobei das nicht magensaftresistente Polymer ausgewählt ist aus Polyvinylpyrrolidon, Poly(vinylpyrrolidon/vinylacetat), Polyvinylcaprolactam/Polyvinylacetat/Polyethylenglycol-Pfropfcopolymer, Polyethylenglycol/Polyvinylalcohol-Pfropfcopolymer, Poly(ethylenoxid/propylenoxid), Macrogolglycerolhydroxystearat, Polyethylenglycol und einem wasserlöslichen neutralen oder anionischen Polysaccharid, und vorzugsweise ein wasserlösliches neutrales oder anionisches Polysaccharid ist.

10. Granulat gemäß Anspruch 9, wobei das wasserlösliche neutrale oder anionische Polysaccharid ausgewählt ist aus Gummi, Pektin, Dextran, Dextrin, einem Celluloseether, vorverkleisterter Stärke und einem Stärkeether, und vorzugsweise ein Celluloseether wie Hydroxypropylcellulose ist.

11. Granulat gemäß einem der vorstehenden Ansprüche, wobei das Gemisch ein Antioxidationsmittel enthält, wobei das Antioxidationsmittel vorzugsweise ausgewählt ist aus Butylhydroxytoluol (BHT), Butylhydroxyanisol (BHA), Natrium- oder Kaliummetabisulfit, Natriumbisulfit, Natriumsulfit, Natriumthiosulfat, Cystein, Acetylcystein, Methionin, Glutathion, Natriumhydroxymethansulfinat, Ascorbinsäure, Natriumascorbat, Ascorbylpalmitat, Tocopherol, Tocopherolsuccinat, Tocopherolpolyethylenglycolsuccinat (TPGS) und Propylgallat, und besonders bevorzugt Propylgallat ist.

12. Granulat gemäß einem der vorstehenden Ansprüche, wobei das Gemisch einen monomeren Weichmacher enthält, wobei der monomere Weichmacher vorzugsweise ausgewählt ist aus Triethylcitrat, Triacetin, Dibutylsebacat, Diethylphthalat, Glycerolmonostearat, Glycerin und Propylenglycol, und besonders bevorzugt Triethylcitrat ist.

13. Granulat gemäß einem der vorstehenden Ansprüche, wobei das Gemisch einen Zuckeralkohol enthält, wobei der Zuckeralkohol vorzugsweise ausgewählt ist aus Xylitol, Sorbitol, Mannitol und Maltitol, und besonders bevorzugt Xylitol ist.

14. Granulat gemäß einem der vorstehenden Ansprüche, wobei das Granulat aus Posaconazol, dem magensaftresistenten Polymer, dem nicht magensaftresistenten Polymer, dem monomeren Weichmacher, dem Zuckeralkohol und gegebenenfalls dem Antioxidationsmittel besteht.

15. Granulat gemäß einem der vorstehenden Ansprüche, wobei das Gemisch mittels Schmelzextrusion hergestellt ist.

16. Verfahren zur Herstellung eines Granulats gemäß einem der Ansprüche 1 bis 15, umfassend die Schritte:
i) Herstellen eines Gemischs, enthaltend Posaconazol, das magensaftresistente Polymer und gegebenenfalls das nicht magensaftresistente Polymer, wobei das Gewichtsverhältnis des magensaftresistenten Polymers zu Posaconazol im Bereich von 5:1 bis 1:1 liegt,
ii) Unterziehen des im Schritt (i) erhaltenen Gemischs einer Schmelzextrusion bei einer Temperatur von 100-170°C, vorzugsweise 100-160°C und mehr bevorzugt 120-150°C,
iii) Mahlen des im Schritt (ii) erhaltenen Extrudats in einem Ausmaß, so dass mindestens 10 Gew.-%, vorzugsweise mindestens 20 Gew.-%, mehr bevorzugt mindestens 30 Gew.-% und noch mehr bevorzugt mindestens 40 Gew.-% des Granulats, aber nicht mehr als 80 Gew.-%, vorzugsweise nicht mehr als 70 Gew.-%, mehr bevorzugt nicht mehr als 60 Gew.-% und sogar noch mehr bevorzugt nicht mehr als 55 Gew.-%, eine Partikelgröße von 250 µm oder mehr aufweisen, bestimmt mittels Siebanalyse, wobei die Mahltemperatur nicht höher als 50°C ist, vorzugsweise nicht höher als 40°C.

17. Verfahren gemäß Anspruch 16, wobei die Drehgeschwindigkeit der Schnecke bei der Schmelzextrusion im Bereich von 150 UpM bis 300 UpM liegt, vorzugsweise im Bereich von 200 UpM bis 265 UpM, mehr bevorzugt im Bereich von 225 UpM bis 250 UpM.

18. Magensaftresistente, gegebenenfalls filmbeschichtete Tablette, umfassend das Granulat gemäß einem der Ansprüche 1 bis 15 und einen pharmazeutischen Hilfsstoff.

19. Magensaftresistente, gegebenenfalls filmbeschichtete Tablette gemäß Anspruch 18, wobei der pharmazeutische Hilfsstoff aus einem Füllmittel, Bindemittel, Sprengmittel, Fließregulierungsmittel und Schmiermittel ausgewählt ist.

20. Magensaftresistente, gegebenenfalls filmbeschichtete Tablette gemäß Anspruch 19, wobei Posaconazol in einem Gemisch, enthaltend Poly(methacrylsäure/ethylacrylat), Triethylcitrat, Hydroxypropylcellulose, Xylitol und Propylgallat, molekular dispergiert ist.

21. Magensaftresistente, gegebenenfalls filmbeschichtete Tablette gemäß Anspruch 19, wobei Posaconazol in HPMCAS oder in einem Gemisch aus HPMCAS und Hydroxypropylcellulose molekular dispergiert ist.

## Revendications

1. Granules comprenant du posaconazole dispersé de manière moléculaire dans un mélange contenant un polymère entérique et facultativement un polymère non entérique, dans lesquels le rapport pondéral du polymère entérique au posaconazole est dans la plage de 5:1 à 1:1, et dans lesquels au moins 10 % en poids des granules, mais pas plus de 80 % en poids, présentent une taille de particule de 250 µm ou plus, telle que déterminée par analyse granulométrique.

2. Granules selon la revendication 1, dans lesquels au moins 20 % en poids des granules, mais pas plus de 70 % en poids, présentent une taille de particule de 250 µm ou plus, telle que déterminée par analyse granulométrique.

3. Granules selon l'une quelconque des revendications précédentes, dans lesquels au moins 30 % en poids des granules, mais pas plus de 60 % en poids, présentent une taille de particule de 250 µm ou plus, telle que déterminée par analyse granulométrique.

4. Granules selon l'une quelconque des revendications précédentes, dans lesquels au moins 40 % en poids des granules, mais pas plus de 55 % en poids, présentent une taille de particule de 250 µm ou plus, telle que déterminée par analyse granulométrique.

5. Granules selon l'une quelconque des revendications précédentes, dans lesquels d (0,1) des granules est d'au moins 60 µm, de préférence d'au moins 75 µm, et de manière plus préférée d'au moins 100 µm, et/ou n'est pas supérieure à 250 µm, de préférence pas supérieure à 180 µm, et de manière plus préférée pas supérieure à 130 µm, telle que mesurée par un procédé à sec de diffusion de lumière dynamique.

6. Granules selon l'une quelconque des revendications précédentes, dans lesquels d (0,5) des granules est d'au moins 160 µm, de préférence d'au moins 200 µm, et de manière plus préférée d'au moins 250 µm, et/ou n'est pas supérieure à 500 µm, de préférence pas supérieure à 400 µm, et de manière plus préférée pas supérieure à 320 µm, telle que mesurée par un procédé à sec de diffusion de lumière dynamique.

7. Granules selon l'une quelconque des revendications précédentes, dans lesquels d (0,9) des granules est d'au moins 300 µm, de préférence d'au moins 400 µm, et de manière plus préférée d'au moins 480 µm, et/ou n'est pas supérieure à 1000 µm, de préférence pas supérieure à 800 µm, et de manière plus préférée pas supérieure à 600 µm, telle que mesurée par un procédé à sec de diffusion de lumière dynamique.

8. Granules selon l'une quelconque des revendications précédentes, dans lesquels le polymère entérique est l'acétate succinate d'hypromellose (HPMCAS) et/ou un dérivé d'acide polyméthacrylique choisi parmi le poly(acide méthacrylique/méthacrylate de méthyle) et le poly(acide méthacrylique/acrylate d'éthyle), et de préférence est le poly(acide méthacrylique/acrylate d'éthyle).

9. Granules selon l'une quelconque des revendications précédentes, dans lesquels le polymère non entérique est choisi parmi la polyvinylpyrrolidone, le poly(vinylpyrrolidone/ acétate de vinyle), un copolymère greffé de polyvinylcaprolactame/polyvinylacétate/polyéthylène glycol, un copolymère greffé de polyéthylène glycol/alcool polyvinylique, le poly(oxyde d'éthylène/oxyde de propylène), l'hydroxystéarate de macrogolglycérol, le polyéthylène glycol et un polysaccharide neutre ou anionique hydrosoluble, et est de préférence un polysaccharide neutre ou anionique hydrosoluble.

10. Granules selon la revendication 9, dans lesquels le polysaccharide neutre ou anionique soluble dans l'eau est choisi parmi une gomme, la pectine, le dextrane, la dextrine, un éther de cellulose, de l'amidon prégélatinisé et un éther d'amidon, et est de préférence un éther de cellulose tel que l'hydroxypropylcellulose.

11. Granules selon l'une quelconque des revendications précédentes, dans lesquels le mélange contient un antioxydant, dans lequel l'antioxydant est de préférence choisi parmi l'hydroxytoluène butylé (BHT), l'hydroxyanisole butylé (BHA), le métabisulfite de sodium ou de potassium, le bisulfite de sodium, le sulfite de sodium, le thiosulfate de sodium, la cystéine, l'acétylcystéine, la méthionine, le glutathion, le formaldéhyde sulfoxylate de sodium, l'acide ascorbique, l'ascorbate de sodium, le palmitate d'ascorbyle, le tocophérol, le succinate de tocophérol, le succinate de polyéthylène glycol de tocophérol (TPGS) et le gallate de propyle, et le gallate de propyle est particulièrement préféré.

12. Granules selon l'une quelconque des revendications précédentes, dans lesquels le mélange contient un plastifiant monomère, dans lesquels le plastifiant monomère est de préférence choisi parmi le citrate de triéthyle, la triacétine, le sébacate de dibutyle, le phtalate de diéthyle, le monostéarate de glycéryle, la glycérine et le propylène glycol, et le citrate de triéthyle est particulièrement préféré.

13. Granules selon l'une quelconque des revendications précédentes, dans lesquels le mélange contient un alcool de sucre, dans lesquels l'alcool de sucre est de préférence choisi parmi le xylitol, le sorbitol, le mannitol et le maltitol, et le xylitol est particulièrement préféré.

14. Granules selon l'une quelconque des revendications précédentes, dans lesquels les granules sont constitués de posaconazole, du polymère entérique, du polymère non entérique, du plastifiant monomère, de l'alcool de sucre et facultativement de l'antioxydant.

15. Granulés selon l'une quelconque des revendications précédentes, dans lesquels le mélange est préparé par extrusion à chaud.

16. Procédé de préparation des granulés selon l'une quelconque des revendications 1 à 15, comprenant les étapes suivantes consistant à :
(i) préparer un mélange contenant du posaconazole, le polymère entérique et facultativement le polymère non entérique, dans lequel le rapport pondéral du polymère entérique au posaconazole est dans la plage de 5:1 à 1:1,
(ii) soumettre le mélange obtenu à l'étape (i) à une extrusion à chaud à une température de 100 à 170°C, de préférence de 100 à 160°C, de manière plus préférée de 120 à 150°C,
(iii) broyer l'extrudat obtenu à l'étape (ii) dans la mesure où au moins 10 % en poids, de préférence au moins 20 % en poids, de manière plus préférée au moins 30 % en poids, et de manière encore plus préférée au moins 40 % en poids des granulés, mais pas plus de 80 % en poids, de préférence pas plus de 70 % en poids, de manière plus préférée pas plus de 60 % en poids, et de manière encore plus préférée pas plus de 55 % en poids, présentent une taille de particules de 250 µm ou plus, telle que déterminée par analyse granulométrique, dans lequel la température de broyage n'est pas supérieure à 50°C, de préférence pas supérieure à 40°C.

17. Procédé selon la revendication 16, dans lequel la vitesse de rotation de la vis pendant l'extrusion à chaud est dans la plage de 150 tr/min à 300 tr/min, de préférence de 200 tr/min à 265 tr/min, de préférence de 225 tr/min à 250 tr/min.

18. Comprimé gastro-résistant, facultativement enrobé d'un film, comprenant les granules selon l'une quelconque des revendications 1 à 15 et un excipient pharmaceutique.

19. Comprimé gastro-résistant, facultativement enrobé d'un film selon la revendication 18, dans lequel l'excipient pharmaceutique est choisi parmi un diluant, un liant, un désintégrant, un agent de glissement et un lubrifiant.

20. Comprimé gastro-résistant, facultativement enrobé d'un film selon la revendication 19, dans lequel le posaconazole est dispersé de manière moléculaire dans un mélange contenant du poly(acide méthacrylique/acrylate d'éthyle), du citrate de triéthyle, de l'hydroxypropylcellulose, du xylitol et du gallate de propyle.

21. Comprimé gastro-résistant, facultativement enrobé d'un film selon la revendication 19, dans lequel le posaconazole est dispersé de manière moléculaire dans du HPMCAS ou dans un mélange de HPMCAS et d'hydroxypropylcellulose.
